(19) Europäisches Patentamt European Patent Office Office européen des brevets

(11) **EP 4 247 245 B1**

(12) **EUROPEAN PATENT SPECIFICATION**

(45) Date of publication and mention of the grant of the patent:
**01.01.2025 Bulletin 2025/01**

(21) Application number: **21823361.7**

(22) Date of filing: **12.11.2021**

(51) International Patent Classification (IPC):
**A61B 5/021** *(2006.01)*   **A61B 5/00** *(2006.01)*

(52) Cooperative Patent Classification (CPC):
**A61B 5/02125; A61B 5/7214; A61B 5/7221; A61B 5/7246; A61B 5/725; A61B 5/7264; A61B 5/7253**

(86) International application number:
**PCT/IB2021/060508**

(87) International publication number:
**WO 2022/106970 (27.05.2022 Gazette 2022/21)**

(54) **METHOD AND SYSTEM FOR REAL-TIME MEASUREMENT OF A SPHYGMIC WAVE VELOCITY (PWV)**

VERFAHREN UND SYSTEM ZUR ECHTZEITMESSUNG DER GESCHWINDIGKEIT EINER SPHYGMISCHEN WELLE (PWV)

PROCÉDÉ ET SYSTÈME POUR LA MESURE EN TEMPS RÉEL D'UNE VITESSE D'ONDE SPHYMIQUE (VITESSE DE L'ONDE DE POULS)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.11.2020 IT 202000027846**

(43) Date of publication of application:
**27.09.2023 Bulletin 2023/39**

(73) Proprietors:
• **Politecnico di Torino**
  **10129 Torino (IT)**
• **Università degli Studi di Torino**
  **10124 Torino (IT)**

(72) Inventors:
• **BURAIOLI, Irene**
  **10129 Torino (IT)**
• **DEMARCHI, Danilo**
  **10129 Torino (IT)**
• **MILAN, Alberto**
  **10124 Torino (IT)**
• **VEGLIO, Franco**
  **10124 Torino (IT)**
• **LEONE, Dario**
  **10124 Torino (IT)**

• **VALLELONGA, Fabrizio**
  **10124 Torino (IT)**

(74) Representative: **Fiume, Orazio et al**
  **Praxi Intellectual Property S.p.A.**
  **Corso Vittorio Emanuele II, 3**
  **10125 Torino (IT)**

(56) References cited:
**US-A1- 2017 238 817**

• **BURAIOLI IRENE ET AL: "A New Noninvasive System for Clinical Pulse Wave Velocity Assessment: The Athos Device", IEEE TRANSACTIONS ON BIOMEDICAL CIRCUITS AND SYSTEMS, IEEE, US, vol. 15, no. 1, 9 February 2021 (2021-02-09), pages 133 - 142, XP011846913, ISSN: 1932-4545, [retrieved on 20210330], DOI: 10.1109/TBCAS.2021.3058010**
• **NABEEL P M ET AL: "Local Pulse Wave Velocity: Theory, Methods, Advancements, and Clinical Applications", IEEE REVIEWS IN BIOMEDICAL ENGINEERING, IEEE, USA, vol. 13, 26 July 2019 (2019-07-26), pages 74 - 112, XP011766934, ISSN: 1937-3333, [retrieved on 20200117], DOI: 10.1109/RBME.2019.2931587**

• CHIU Y C ET AL: "Determination of pulse wave velocities with computerized algorithms", AMERICAN HEART JOURNAL, ELSEVIER, AMSTERDAM, NL, vol. 121, no. 5, 1 May 1991 (1991-05-01), pages 1460 - 1470, XP022900082, ISSN: 0002-8703, [retrieved on 19910501], DOI: 10.1016/0002-8703(91)90153-9

## Description

## Field of the invention

The present invention relates to the field of bio-signal measurements relating to the cardiovascular system.

## State of the art

Numerous studies have shown that arterial vascular stiffness is a predictor of cardiovascular risk and mortality, as it provides an indicator about the state of health of the arteries and allows the early identification of organ damage even if still clinically asymptomatic.

It is possible to estimate arterial stiffness in a non-invasive way by measuring the speed of the sphygmic wave or pulse (pulse wave velocity, PWV).

The PWV trend measurement is achieved by measuring the propagation time between two sites, typically the neck (carotid artery) and the thigh (femoral artery). Then, by considering the distance between the two measurement sites and dividing it by the pulse propagation time, the propagation speed (PWV) is obtained. Exemplary PWV estimation systems are disclosed by NABEEL P M ET AL: "Local Pulse Wave Velocity: Theory, Methods, Advancements, and Clinical Applications",IEEE REVIEWS IN BIOMEDICAL ENGINEERING, IEEE, USA, vol. 13, 26 July 2019.

Although this parameter is considered relevant in the evaluation of cardiovascular risk, it is hardly taken into consideration due to the difficulty in carrying out the measurement and the post-processing time that this requires. According to the known art, the evaluation of PWV is performed off-line, that is to say a certain time after the acquisition of the signals relating to the pulse waves. These signals are generated by a transducer, typically a pressure sensor placed in contact with the patient's skin near an artery to measure the propagation time of the heartbeat.

Current PWV parameter measurement tools have limitations, such as:

- long measurement times, and long waiting times for obtaining estimations,
- poor uniformity in the estimation of values and presence of discrepancies for high PWV values (> 10 m/s). Furthermore, in some cases the signal acquisition procedure is laborious and highly dependent on the operator, with the well-founded risk of unreliability.

For all the devices currently on the market, in fact, a long acquisition is required until the pulse signals necessary to measure the PWV are stable for a certain predetermined time period. And only after the acquisition, the signals are processed, but in this phase, the medical operator cannot intervene to change the position of the transducers and, above all, does not have control of any oscillations in the PWV trend, intrinsically related to the state of the cardiovascular system. Therefore, if for example the signals have a low S/N ratio, then it is necessary to repeat, blindly, the acquisition procedure until the acquisition process is considered satisfactory.

To evaluate the transit time (PTT) elapsed between the propagation of the pulse wave from the carotid to the femoral arterial, it is necessary to extrapolate a point on both signals that allows to identify the exact passage. Generally, the aim is to recognize the "foot" of the sphygmic wave for each heartbeat. This method is defined as the "intersecting tangent algorithm" and includes the following steps:

- determination of the maximum point of first derivative MD on the rising front of the sphygmic wave Sig;
- application of the Tan tangent to the rising edge at the point of maximum first derivative MD,
- identification of the relative minimum MR immediately to the left of the tangent,
- tracing of a horizontal axis passing through this minimum until it intersects the tangent,
- projection of the intersection point on the signal Sig.

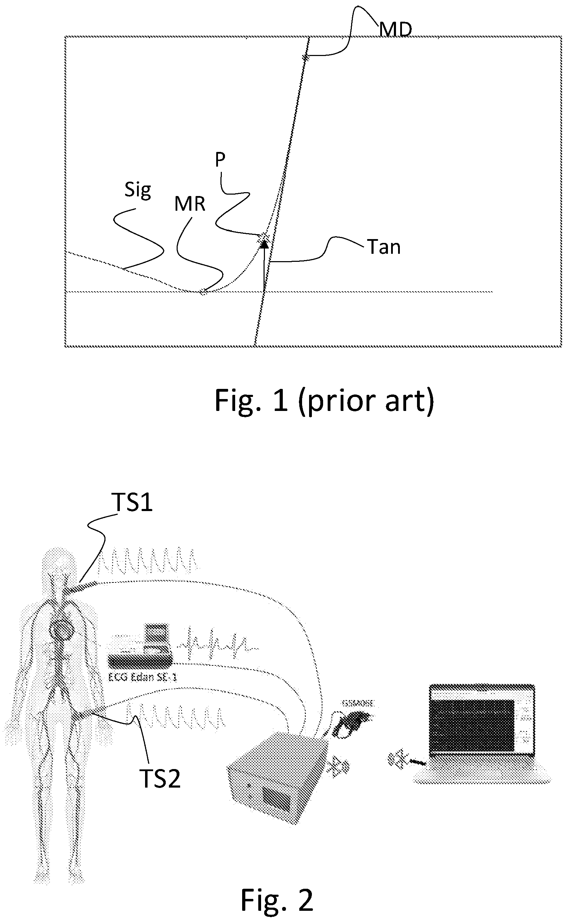

Figure 1 shows the projection P of the intersection point on the signal Sig, by convention defining the foot P of the wave. The projection of the intersection on the signal is indicated by an arrow that indicates, in fact, the foot P.

At present there are no known methods, which allow the above steps to be carried out in real time.

If not specifically excluded in the following detailed description, what is described in this chapter is to be considered as an integral part of the detailed description.

## Summary of the invention

The invention is defined by appended claims 1-15.

The purpose of the present invention is to provide a real-time monitoring system of the PWV trend.

The basic idea of the present invention is to obtain a passband-filtered digital signal from which to identify relative minima (event starter), which act as reference points, on the basis of which the unfiltered or high-pass filtered numeric signal is windowed for perform the detection of the foot of the sphygmic wave by means of the per se known algorithm of the intersecting tangent algorithm.

The digital signal is analyzed by means of sampling segments, little by little the sensors generate the relating electrical signals, being them analog or digital ones.

The width of the segments is preferably fixed and predetermined. However, it can be expected to adapt the width of the segments, as the algorithm synchronizes itself with the heart rate and the width stabilizes itself.

For clarity, "current segment" is defined as the last segment, in chronological order, acquired.

According to the present invention, the search for the foot of the sphygmic wave is performed in a sliding observation window of variable amplitude so that the entire sphygmic pulse is always contained in the observation window. According to a first aspect, when an impulse is not completely contained in the current segment, the final portion of the current segment containing the initial portion of the incomplete impulse is attached to the "next/future current" segment. Advantageously, no sphygmic impulse is omitted due to lack of synchronism between the segmentation operation and the heart rate of the subject under investigation.

Thanks to the present invention, the signal acquisition process is self-synchronized with the patient's heart rate, allowing to perform a PWV analysis in real time and above all not operator-dependent.

According to a first preferred variant, when a pulse is not completely contained in the current segment, it is in any case analyzed in real time to search for relative minima and subsequently, the final portion of the current segment containing the initial part of the pulse is attached to the next current segment and only then the foot of the pulse is identified among the relative minima previously identified. According to a further preferred aspect, substantially real-time operations are performed on the available pulse portion, even if it is not possible to complete the algorithm of the intersecting tangent in the current segment as the entire sphygmic pulse is not yet available.

In addition, when the signal has many artifacts, the search for relative minima among the candidates of the wave foot, before attaching the available portion of the window to the next current segment, turns out to be more advantageous in terms of the quality of the signal to be analysed, in view of the distortions brought about by the filtering in the initial part of the segment.

According to a second preferred variant, the intersecting tangent algorithm is entirely executed only when the pulse is complete, that is, only after the segment portion containing an initial portion of the pulse is attached to the next current segment. Obviously, this variant provides for a faster calculation of the processing unit, but allows to avoid identifying the relative minima in the previously identified window.

Advantageously, the possibility of measuring the PWV trend in real time allows to considerably expand the possible applications to allow for drastically modifying the known diagnostic protocols.

More specifically, the present invention allows the operator to evaluate the quality of the acquisitions in real time, so as to be able to promptly intervene on the positioning of the sensors.

The invention is defined by the appended claims.

## Brief description of the figures

Further objects and advantages of the present invention will become clear from the following detailed description of an example of its embodiment (and its variants) and from the attached drawings given purely by way of non-limiting explanation, in which:

Figure 1 shows a method, according to the known art, for the recognition of the sphygmic wave;
Figure 2 shows a monitoring system according to the present invention;
Figure 3 shows a segmentation scheme of the acquired data;
Figures 4 - 6 show time diagrams and the search for the foot of a sphygmic wave respectively:

- in a case where the entire sphygmic wave is contained in a current observation segment,
- a portion of the sphygmic wave is contained in the current observation segment, allowing the retrieval of a set of all possible relative minima among which to identify the foot of the wave or pulse according to the intersecting tangent algorithm,
- a portion of the sphygmic wave is contained in the current window but is insufficient to allow any operation;

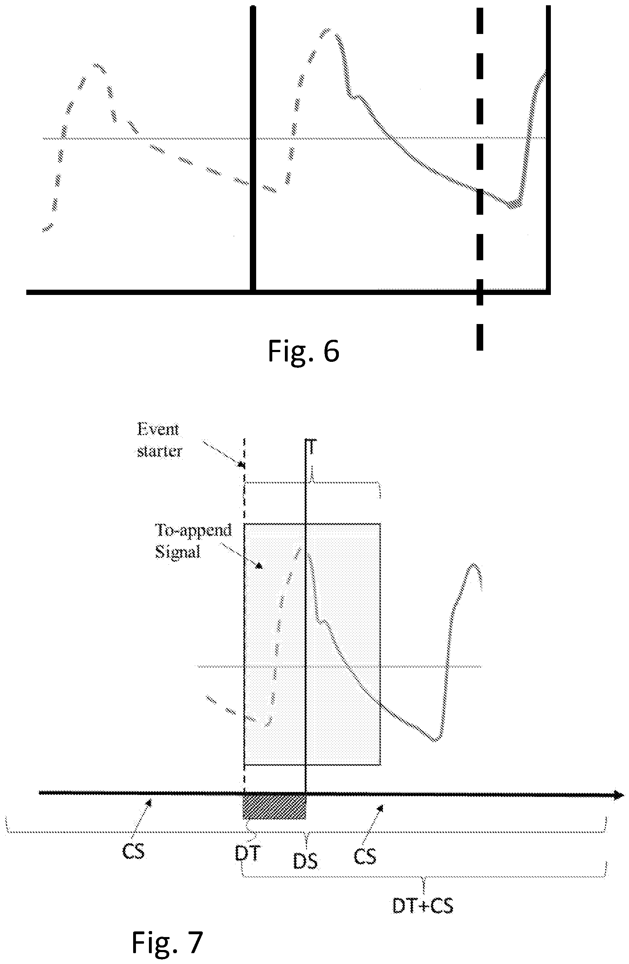

Figure 7 shows an operation of attaching the terminal portion of the previous segment to the current observation segment, expanding its amplitude in order to identify all the feet of the sphygmic wave, even those that fall halfway between two observation segments;

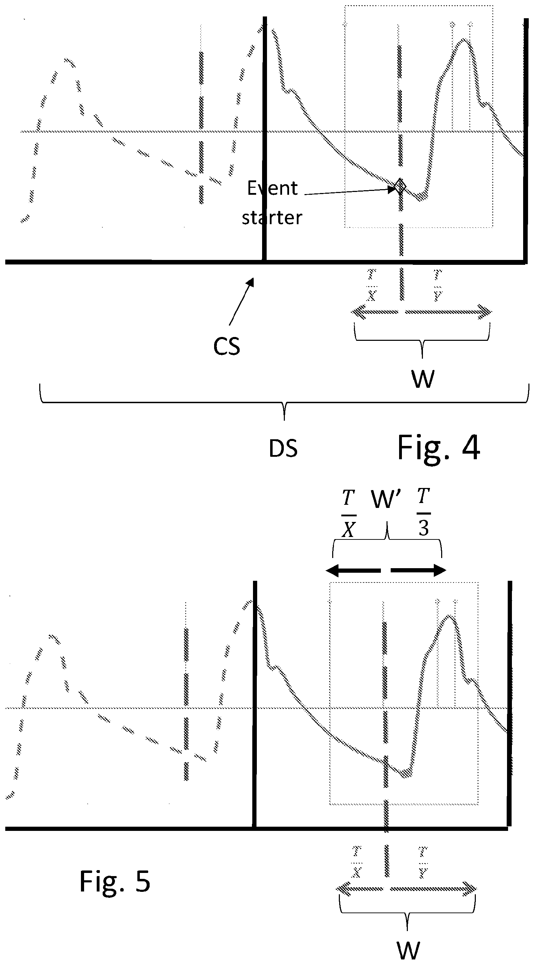

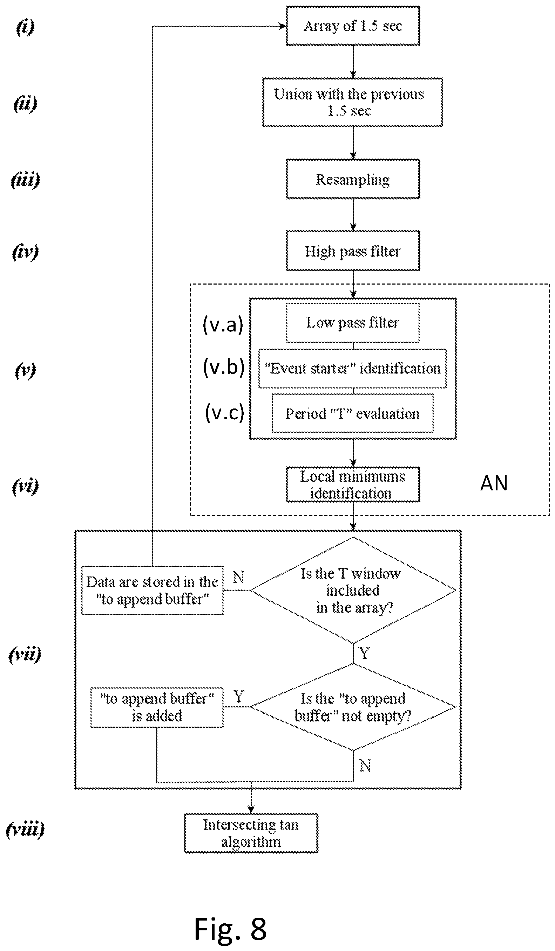

Figure 8 shows a flow chart representative of a preferred variant of the invention corresponding to the example of Figures 5 and 6,

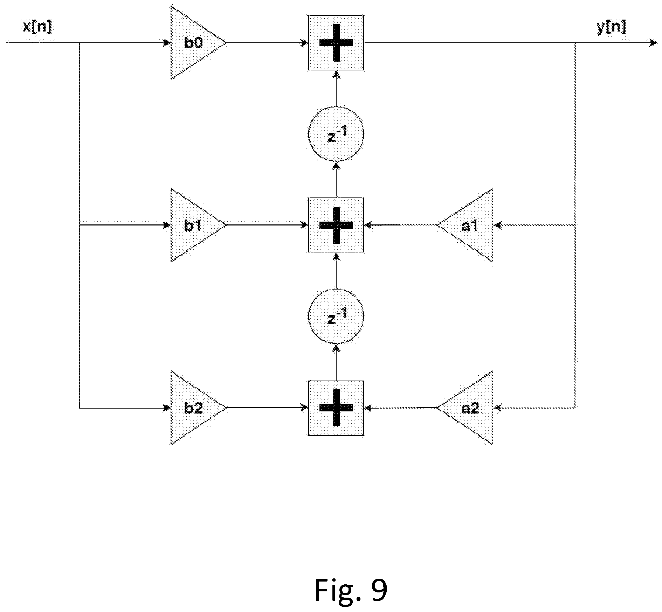

Figure 9 shows a model-base diagram of a biquadratic filter implemented for the high-pass filtering of an acquired sphygmic signal.

The same reference numbers and letters in the figures identify the same elements or components.

In the context of this description, the term "second" component does not imply the presence of a "first" component. These terms are in fact used as labels to improve clarity and should not be understood in a limiting way.

## Description of detailed examples

With reference to Figure 2, two tonometric sensors TS1, TS2 are used to be positioned in the acquisition sites, combined with a CPU processing unit comprising an interface for the acquisition of the signals generated by the tonometric sensors and preferably an interface for the simultaneous acquisition of electrocardiographic signals. Electrocardiographic signals are absolutely not necessary when taking measurements at two acquisition sites using the aforementioned two sensors TS1 and TS2.

Conversely, electrocardiographic signals are essential only when a single tonometric sensor is used.

The purpose is to compare the time lag of two wave feet of the same heart pulse to determine the PWV.

It is worth noting that for the purposes of the present description the concept of heart impulse can be confused with the concept of sphygmic wave which depends on it. According to the present invention, a time window of 1.5 seconds is considered, at least initially. This choice allows the comfortable observation of at least one cardiac event even in the case of bradycardic subjects, in fact 40bpm corresponds to a heartbeat every 1.5 seconds.

Preferably, to obtain an acceptable resolution, a time resolution of 1.5 ms has been chosen, the sampling frequency of which corresponds to 680Hz, which, in turn, corresponds to a multiple of the sampling frequency of the tonometric sensors, which are characterized for a sampling frequency of the order of 170 Hz; in this case it is therefore necessary to carry out a conversion of the sampling frequency, restoring the signals of the tonometric sensors to a frequency of 680 Hz.

With an observation period of 1.5 seconds, the samples amount to 1020. Therefore, vectors (arrays) of 1020 values are generated, including the last acquired is indicated with the label CS.

The segmentation of the signal acquired every 1.5 seconds is strictly preferential. As this time interval allows a convenient on-screen updating of the vascular parameter. However, it is necessary to underline how the same algorithm can also work with different temporal segmentations, up to having segments capable of containing a single cardiac pulse with the consequent updating of the PWV parameter at each pulse. In fact, the present invention is totally adaptable on the basis of parameters that can be set by the operator or on the basis of automatic adaptations.

In particular, in the case of updating the parameter every beat, it is foreseen that the segmentation of the signal is initially fixed, for example of 1.5 seconds as described above. After analyzing, for example, 10 seconds of signal with segments of pre-ordered amplitude, an average cardiac period is calculated:

- the size of the segment is adapted to this period
- the first sample of each segment is moved so that the segment contains an entire heart pulse.

Advantageously, in this way, the algorithm automatically synchronizes itself with the heart rate and the dynamic window thus becomes automatic.

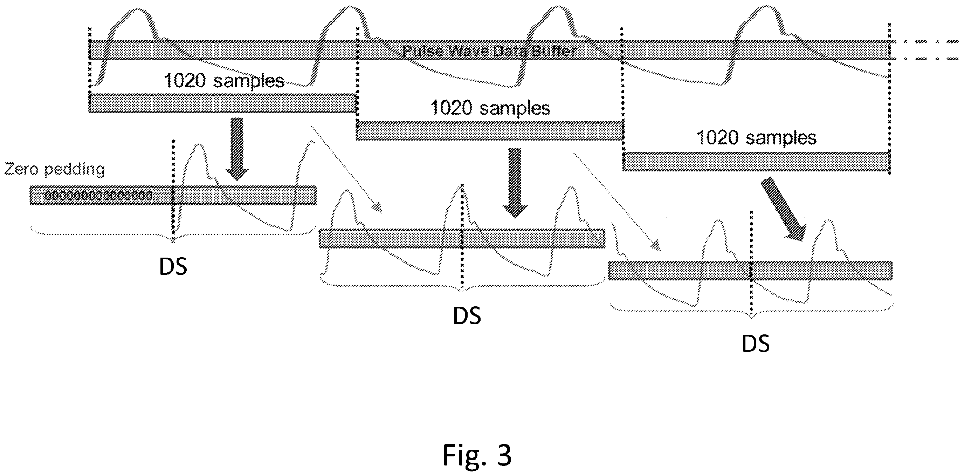

According to the present invention, two contiguous segments are considered, indicated as a whole with the label DS, in particular, the current segment CS is considered on the right and the segment that precedes it on the left, assuming that the time axis is right oriented. Thus, vectors with 2040 samples are obtained, hereinafter referred to as "double segment" DS, as shown in figure 3, in which the segments indicated with the words "Pulse Wave data Buffer" are shown, while "samples" are indicated in the English language as "Samples". This aspect essentially impacts on the filtering techniques described below, to carry out an accurate analysis of the acquired signals. It is not excluded that further and different filtering techniques can be identified that make it possible to avoid an analysis on a double segment. Alternatively, it may not be necessary to resort to the double segment with sensors having sampling frequencies much higher than those currently available.

In the case of the first acquisition segment, to obtain the double segment, a virtually previous segment is added, with all values set to zero.

The signal contained in the double segment is preferably interpolated by means of a cubic spline to be sampled at 2040 Hz or higher.

Preferably, the third degree interpolating polynomial is chosen so as to ensure continuity of the second derivative order.

Subsequently, the signal is high pass filtered with a frequency sufficient to eliminate any offset between the pulses, for example 0.5 Hz. The cutoff frequency of 0.5 Hz has been selected because it is a frequency close enough to the zero frequency (continous), but high enough to allow rapid processing of the signal being filtered, taking into account the scope of obtaining a real-time analysis of the signals.

Subsequently, the signal obtained is low-pass filtered with a frequency of 2 Hz to try to extract the basic pulsatile component of the sphygmic signals, in order to identify single cardiac events in the signal segment considered, without being misled by atypical events (artifacts) or noise. After filtering, the relative minima necessary to identify, at least approximately, the single cardiac events, contained in the double segment not filtered or only high-pass filtered, are identified.

These relative minima, hereinafter referred to as "event starter", however, do not correspond to the foot P of the sphygmic wave necessary for the calculation of the PWV, nor do they have any bearing on the relative minimum MR immediately to the left of the tangent Tan as shown in figure 1, but have the purpose of guiding the composition of portions of contiguous segments and/or the synchronization of the segmentation procedure, in order to analyze complete cardiac impulses and to be able to fully apply the intersecting tangent algorithm.

In other words, the identification of the event starter allows to scroll the observation window in the time trace acquired in order to observe an entire impulse at a time. These event starters are in fact useful for determining the approximate period T between two consecutive cardiac impulses and are useful for identifying the relative minima among which MR will be identified, as the minimum value closest to the tangent Tan.

A first observation window W is constructed, as indicated in figure 4. This window is applied to the unfiltered or high-pass filtered signal and straddles the dashed vertical line passing through an event starter identified in the corresponding temporal portion of the same signal, but band pass filtered.

It is worth pointing out that in figure 4, it does not appear to be the absolute minimum in the window W, since the signal of figure 4 is unfiltered or only high-pass filtered and therefore includes all the high frequency artifacts.

However, it is preferred to perform the windowing and identification of the foot of the sphygmic wave on the high pass filtered signal, as the offset introduces instability in the analysis, although it is not excluded that this instability can be eliminated with other techniques other than high-pass filtering.

This window extends to the left of the dashed vertical line for a left sub-interval T/X, which can be a reasonably small fraction of T, which can vary between T/10 and T/3. It could also have a fixed width, but it is not recommended. The right sub-interval T/Y is wider than the left one because it is intended to reasonably include the wave front P on which to identify the tangent according to the intersecting tangent algorithm. It can be between 2T/3 and T.

Advantageously, the observation window W has an amplitude that depends on the cardiac period T, adapting itself to the signals generated by the patient under investigation. According to the invention, attention is focused essentially on the current segment CS, that is, on the right segment of each double segment DS, as the relative samples are acquired. Two conditions can occur:

> a) the window W is integrally contained in the current segment CS, i.e. the right segment of the double segment of figures 4,
> b) the window W is only partially contained in the current segment, continuing in the future current segment.

In the first case it is possible to immediately identify the point MD, the tangent Tan and the minimum point immediately preceding (on the left) of the tangent, etcc.

In the second case, you can proceed in two different ways. According to a first preferred variant of the invention, the DT portion of the window W is attached to the next current segment CS and the pulse foot is identified.

According to a second variant, it is checked whether there are the conditions for carrying out pre-processing.

More specifically, with reference to Figure 5, the availability of a window W' is verified which includes a left interval at the event starter of T/X as for W, but on the right it has a sub-interval lower than T/Y, for example T/3.

Although this sub-interval is insufficient to complete the identification of the pulse foot, it is useful for identifying the relative minima, in the non-filtered or only high-pass filtered signal, among which the impulse foot will be identified later.

Also in this case the portion DT of W available is attached to the next current segment CS, but having already stored in a buffer the most probable minima among which the pulse foot is selected later.

This fact makes the present analysis system as contemporary as possible to the acquisition of signals.

If the event starter is very close to the end of the current segment, see figure 6, i.e. the event starter is less than T/3 from the end of the current segment, the calculation of the relative minima is inhibited, postponing the procedure after the step of attaching DT to the next current segment, see figure 7.

In other words, the step of searching for the relative minima and the step of attaching the portion DT to CS can be inverted in terms of the listing order of execution in relation to the circumstances.

Figure 7 shows the procedure for attaching DT to the current "new" current segment. From an operational point of view, it is as the current segment dilates and shrinks in relation to the reciprocal time positioning between heart impulses and segments.

This allows for an observation window that flows "smoothly" over time, although the acquisition of signals occurs in discrete segments.

Figure 8 represents a flow chart of a preferred variant of the present invention. The steps are listed below:

- Step i: acquisition of samples, for example 1020, in a time interval, for example of 1.5 seconds, to define a current segment corresponding to the current observation window CS,
- Step ii: joining the current segment with the immediately preceding segment to form a double DS segment,
- Step iii: interpolation and resampling at higher frequency,
- Step iv: high pass filtering for offset elimination,
- Step v.a: low pass filtering,
- Step v.b: identification of event starters and
- Step v.c: calculation of the average period T among the identified event starters,
- Step vi: identification of the local minima in window W (or in a portion of it W') identified on the basis of the event starters,
- Step vii: if the observation window T, having an amplitude equal to the period T and extreme left at the last event starter identified, is entirely included in the current observation window CS, then it proceeds to identify the tangent Tan and the foot P of the wave, if instead the window W is only partially included in the current observation segment CS, then it waits for the next current observation segment and attach the DT portion of the window T together with the next current observation segment CS, defining the interval DT + CS. Alternatively, if a portion T' of predetermined width of the observation window T is fully contained in the current observation segment, it is possible to identify and store at least the relative minima among which to select, later the pulse foot; otherwise, this operation is also postponed to the acquisition of the next observation segment CS.

In other words, the solution shown by the diagram in Figure 8 provides for a search for the foot of the impulse that can be performed in one or two steps, as the next segment CS becomes available.

The observation window can be equal to the period or it can be reduced slightly for example equal to 3/4T, therefore the observation window can be confused with the period bearing in mind that between them there is a proportionality such that the window has amplitude between 3/4 of the period T and the whole period T.

Once the feet of the corresponding sphygmic waves in the two measurement sites have been identified for the same cardiac pulse, the relative time lag and the parameter PWV are calculated in a known way.

Although the search and storage of a certain number of minima can increase the computation load, compared to the case in which the minimum MR is searched from right to left, this allows to make the analysis fast and simultaneously with the acquisition of new samples, allowing the updating of the information on the screen almost simultaneously with the acquisition of the new segments.

For the purposes of this description it must be clear that an analysis leading to an update every 3 seconds would still be considered in real time, as it would still allow the doctor to immediately detect an incorrect positioning of the sensors.

Some particularly important implementation details are now described.

On **the stability of the measures** According to another aspect, the percentage standard deviation is evaluated on the last 10 values of the PWV parameter and when this is less than 5% then the PWV parameter is more stable. In other words, the standard deviation allows the operator to evaluate the correctness of the PWV measurement in real time. When this standard deviation value is greater, it is the operator's responsibility to accept the current PWV value or intervene, for example, on the repositioning of the sensors.

Another parameter that is believed to ensure greater reliability of the measurements is the signal-to-noise ratio of the signals acquired by the sensors. According to a preferred variant of the invention, the system comprises a device on which the signals acquired by the sensors are shown. These signals are zoomed only when the peak-to-peak amplitude of the extrapolated pulse waves is greater than a certain threshold to indicate greater reliability of the signal acquisition and therefore of the PWV calculation.

**On the reporting method**

According to another aspect, a report is generated containing all the values of the differential propagation times PTT between the two sites and the corresponding PWV values in addition to the final PWV value, which is obtained as an average on the PWV values whose corresponding PTTs result within an interval centered on the average value of the PTT extracted over a predetermined time window. Preferably, this time window is selected on the basis of a relative greater stability of the measurements as described above.

**Filtering**

One of the problems that had to be faced in the realization of the present invention is the filtering of the signal acquired by means of the tonometric sensors.

To ensure that there is no distortion on the high pass filtered signal a fourth order biquadratic filter has been devised, shown with the help of Figure 8, in which each stage implements a linear recursive IIR (infinite impulse response) filter of the second order using the following equation:

$$y[n] \; = \; b0 \, * \, x[n] \; + \; d1$$

$$d1 \; = \; b1 \, * \, x[n] \; + \; a1 \, * \, y[n] \; + \; d2$$

$$d2 \; = \; b2 \, * \, x[n] \; + \; a2 \, * \, y[n]$$

Where

- n represents the index of the discrete sample considered,
- d1, d2 are state variables composed of feedforward coefficients (b0, b1, b2) and feedback coefficients (a1, a2) which delineate the filter mask,
- x and y represent respectively the input signal and the output signal.

More specifically, according to this preferred method of signal filtering, the signal undergoes the following steps:

- Inversion,
- First filtering,
- Inversion,
- Second filtering.

Both the first filtering and the second filtering

operate in the same way in the sense that if it is intended to filter high pass the coefficients a1, a2, b1, b2 are chosen by the person skilled in the art in order to obtain a high pass filtering. The same criterion applies in the case of low pass filtering. Obviously, it is known to those skilled in the art that band-pass filtering can be obtained by first operating a high-pass (or low-pass) filtering and subsequently a low-pass (or high-pass) filtering.

The double inversion with the two intermediate filtering stages ensures the zeroing of any distortion introduced by the filtering operation.

This method is useful in any situation it is necessary to filter both low-pass and high-pass a signal sampled with short-length segments.

Indeed, the fact of considering double segments guarantees two relevant aspects:

- A sufficient length to avoid excessive distortions introduced by the filtering and
- An acceptable continuity of the signal, which is guaranteed precisely by considering the segment immediately preceding the current one.

It was found that the identification of event starts is particularly reliable when this filtering technique is used. Then taking into account that the identification of event starts allows to narrow down the analysis of the signal in order to identify

- The relative minima, the tangent and then the closest and preceding minimum (temporally) the tangent between the relative minima previously identified or
- The tangent and then the closest relative minimum preceding (temporally) the tangent.

The present invention can be advantageously implemented by means of a computer program, which comprises coding means for carrying out one or more steps of the method, when this program is executed on a computer. Therefore, it is intended that the scope of protection extends to said computer program and further to computer readable means comprising a recorded message, said computer readable means comprising program coding means for carrying out one or more steps of the method, when said program is run on a computer. The invention is defined by the appended claims.

**Claims**

1. Real-time measurement method of the speed of the sphygmic wave (PWV) acquired as the time lag between the feet of the same sphygmic wave acquired in two different sites of a human body in the form of corresponding electrical signals generated by as many sensors (TS1, TS2) suitable for detecting a pressure impulse at an artery of the human body, the method comprising a process of acquisition (Step i) of the two electrical signals and a simultaneous analysis process (AN) of the two electrical signals in real time, in which the analysis process includes the search (Step vb), for each electrical signal, of the foot (P) of the sphygmic wave in a portion (W) of the unfiltered or high-pass filtered signal (step iv) corresponding to a neighborhood (W) of a relative minimum of the same portion of the band-pass filtered signal and in which said search is performed in two contiguous segments (CS) of the electrical signal by means of an sliding observation window (T), contained in said two contiguous segments, such as to always include an entire sphygmic wave.

2. Method according to claim 1, wherein said sliding observation window has a variable width and proportional to a period calculated on a succession of identified event starters.

3. Method according to claim 1 or 2, wherein said analysis process comprises

   - passband filtering of the signal represented in at least the current segment,
   - identification of the relative minima (event starter) in the signal filtered in the previous step,
   - check if a neighborhood (W) of the event starter is entirely contained in the current segment (CS), if so
   - calculation of the foot of the sphygmic wave in said neighborhood (W) on the unfiltered or filtered high pass signal, otherwise, if this neighborhood (W) is not entirely contained in the current segment
   - attachment of the available portion of the neighborhood (W) to a next current segment (CS) e
   - calculation of the foot (P) of the sphygmic wave in said neighborhood, on the non-filtered or high-pass filtered signal only.

4. Method according to any one of the preceding claims, wherein if the neighborhood (W) is not entirely contained in the current segment, then if a sub-interval (W') of the neighborhood having predetermined amplitude is entirely contained in the current segment, the relative minima are identified and stored so that when the next current segment is available, the immediately preceding (left) minimum (MR) of the tangent (Tan) is selected.

5. Method according to any one of the preceding claims, comprising a step of constructing a segment given by the current segment (CS) and by at least one adjacent segment which precedes it before carrying out the band pass filtering step.

6. Method according to claim 5, wherein said band-pass filtering comprises a high-pass filtering and a low-pass filtering and wherein each of them comprises the implementation of a biquadratic filter of the fourth order.

7. Method according to claim 6, wherein said filtering comprises the following operations of manipulating the signal in succession:

    - Inversion,
    - First filtering,
    - Inversion,
    - Second filtering.

8. Method according to any one of the preceding claims, comprising the step of calculating the PWV parameter on the basis of the distance between a first sensor (TS1) associated with a first site and a second sensor (TS2) associated with a second site, and on the basis of a time lag between the feet of the same impulse detected in the first and second site.

9. Method according to claim 8, further comprising a step of calculating a standard deviation on a moving window of the last values of the PWV parameter and of indicating the measurement as reliable when the standard deviation is less than a predetermined threshold.

10. Method according to any one of the preceding claims, comprising a step of calculating a signal-to-noise ratio of the signals acquired by the sensors (TS1, TS2) and of indicating as reliable when this ratio exceeds a second predetermined threshold.

11. Method according to any one of the preceding claims, wherein the acquisition process begins with segments having an amplitude corresponding to a predetermined length of time and comprises a synchronization step wherein the amplitude and phase of each segment is synchronized on with the pressure signal detected by the sensors, so that only one pressure pulse is contained in a segment.

12. Method according to claim 11, wherein said predetermined duration is 1.5 seconds.

13. Computer program comprising program coding means suitable for carrying out all the steps of any one of claims 1 to 12, when said program is run on a computer operatively connected with said sensors (TS1, TS2) arranged to be associated in two different sites of the body to detect a pressure impulse.

14. Computer readable means comprising a recorded program, said computer readable means comprising program coding means adapted to perform all steps of any one of claims 1 to 12, when said program is run on a computer operatively connected with said sensors (TS1, TS2) arranged to be associated to two different sites of the body to detect a pressure impulse.

15. Real-time measurement system of the sphygmic wave velocity (PWV) comprising a pair of sensors (TS1, TS2) suitable for detecting a pressure impulse in correspondence with an artery of the human body, a processing unit operationally connected with said pair of sensors and configured to acquire respective pressure signals and to sample them according to a succession of samples organized in segments and configured to carry out all the steps of any one of claims 1 to 12.

**Patentansprüche**

1. Verfahren zur Echtzeitmessung der Geschwindigkeit der Blutdruckwelle (PWV), die als Phasendifferenz zwischen den Füßen derselben Blutdruckwelle erfasst wird, die an zwei verschiedenen Stellen eines menschlichen Körpers in Form von entsprechenden elektrischen Signalen erfasst werden, die von ebenso vielen Sensoren (TS1, TS2) erzeugt werden, die zum Erfassen eines Druckimpulses an einer Arterie des menschlichen Körpers geeignet sind, wobei das Verfahren einen Prozess zum Erfassen (Schritt i) der beiden elektrischen Signale und einen Prozess zum gleichzeitigen Analysieren (AN) der beiden elektrischen Signale in Echtzeit umfasst, wobei der Analyseprozess die Suche (Schritt vb) für jedes elektrische Signal des Fußes (P) der Blutdruckwelle in einem Abschnitt (W) des ungefilterten oder hochpassgefilterten Signals (Schritt iv) umfasst, der einer Umgebung (W) eines relativen Minimums desselben Abschnitts des bandpassgefilterten Signals entspricht, und wobei die Suche in zwei aneinandergrenzenden Segmenten (CS) des elektrischen Signals mittels eines gleitenden Beobachtungsfensters (T) durchgeführt wird, das in den beiden aneinandergrenzenden Segmenten enthalten ist, um immer eine gesamte Blutdruckwelle zu enthalten.

2. Verfahren nach Anspruch 1, wobei das gleitende Beobachtungsfenster eine variable Breite aufweist und proportional zu einer Periode ist, die anhand einer Abfolge von identifizierten Ereignisstartern berechnet wird.

3. Verfahren nach Anspruch 1 oder 2, wobei der Analyseprozess Folgendes umfasst:

    - Durchlassbandfilterung des in mindestens dem aktuellen Segment dargestellten Signals,
    - Identifizierung der relativen Minima (Ereignisstarter) im vorherigen Schritt gefilterten Signal,

- Überprüfung, ob eine Umgebung (W) des Ereignisstarters vollständig im aktuellen Segment (CS) enthalten ist, falls ja
- Berechnung des Fußes der Blutdruckwelle in der Umgebung (W) auf dem ungefilterten oder gefilterten Hochpasssignal, andernfalls, wenn diese Umgebung (W) nicht vollständig im aktuellen Segment enthalten ist
- Anfügen des verfügbaren Teils der Umgebung (W) an ein nächstes aktuelles Segment (CS) und
- Berechnung des Fußes (P) der Blutdruckwelle in der Umgebung, nur auf dem ungefilterten oder hochpassgefilterten Signal.

4. Verfahren nach einem der vorhergehenden Ansprüche, wobei, wenn die Umgebung (W) nicht vollständig im aktuellen Segment enthalten ist, dann, wenn ein Teilintervall (W') der Umgebung mit vorgegebener Amplitude vollständig im aktuellen Segment enthalten ist, die relativen Minima identifiziert und gespeichert werden, so dass, wenn das nächste aktuelle Segment verfügbar ist, das unmittelbar vorhergehende (linke) Minimum (MR) der Tangente (Tan) ausgewählt wird.

5. Verfahren nach einem der vorhergehenden Ansprüche, umfassend einen Schritt des Konstruierens eines Segments, das durch das aktuelle Segment (CS) und durch mindestens ein benachbartes Segment gegeben ist, das diesem vorangeht, bevor der Schritt des Bandpassfilterns ausgeführt wird.

6. Verfahren nach Anspruch 5, wobei die Bandpassfilterung eine Hochpassfilterung und eine Tiefpassfilterung umfasst und wobei jede von ihnen die Implementierung eines biquadratischen Filters der vierten Ordnung umfasst.

7. Verfahren nach Anspruch 6, wobei die Filterung die folgenden Schritte zur Manipulation des Signals nacheinander umfasst:

   - Inversion,
   - Erste Filterung,
   - Inversion,
   - Zweite Filterung.

8. Verfahren nach einem der vorhergehenden Ansprüche, umfassend den Schritt des Berechnens des PWV-Parameters auf der Grundlage der Entfernung zwischen einem ersten Sensor (TS1), der mit einem ersten Standort verbunden ist, und einem zweiten Sensor (TS2), der mit einem zweiten Standort verbunden ist, und auf der Grundlage einer Zeitverzögerung zwischen den Füßen desselben Impulses, der an dem ersten und zweiten Standort erfasst wird.

9. Verfahren nach Anspruch 8, ferner umfassend einen Schritt des Berechnens einer Standardabweichung in einem gleitenden Fenster der letzten Werte des PWV-Parameters und des Anzeigens der Messung als zuverlässig, wenn die Standardabweichung kleiner als ein vorgegebener Schwellenwert ist.

10. Verfahren nach einem der vorhergehenden Ansprüche, umfassend einen Schritt zum Berechnen eines Signal-Rausch-Verhältnisses der von den Sensoren (TS1, TS2) erfassten Signale und zum Anzeigen als zuverlässig, wenn dieses Verhältnis einen zweiten vorgegebenen Schwellenwert überschreitet.

11. Verfahren nach einem der vorhergehenden Ansprüche, wobei der Erfassungsprozess mit Segmenten beginnt, die eine Amplitude aufweisen, die einer vorgegebenen Zeitdauer entspricht, und einen Synchronisierungsschritt umfasst, bei dem die Amplitude und Phase jedes Segments mit dem von den Sensoren erfassten Drucksignal synchronisiert wird, so dass in einem Segment nur ein Druckimpuls enthalten ist.

12. Verfahren nach Anspruch 11, wobei die vorgegebene Dauer 1,5 Sekunden beträgt.

13. Computerprogramm mit Programmcodierungsmitteln, die zum Ausführen aller Schritte eines der Ansprüche 1 bis 12 geeignet sind, wenn das Programm auf einem Computer ausgeführt wird, der operativ mit den Sensoren (TS1, TS2) verbunden ist, die so angeordnet sind, dass sie an zwei verschiedenen Stellen des Körpers angeordnet sind, um einen Druckimpuls zu erfassen.

14. Computerlesbares Mittel, das ein aufgezeichnetes Programm umfasst, wobei das computerlesbare Mittel Programmcodierungsmittel umfasst, die angepasst sind, um alle Schritte eines der Ansprüche 1 bis 12 auszuführen, wenn das Programm auf einem Computer ausgeführt wird, der operativ mit den Sensoren (TS1, TS2) verbunden ist, die so angeordnet sind, dass sie mit zwei verschiedenen Stellen des Körpers verbunden sind, um einen Druckimpuls zu erfassen.

15. Echtzeit-Messsystem der Blutdruckwellengeschwindigkeit (PWV), das ein Paar Sensoren (TS1, TS2) umfasst, die geeignet sind, einen Druckimpuls in Übereinstimmung mit einer Arterie des menschlichen Körpers zu erfassen, eine Verarbeitungseinheit, die operativ mit dem Paar Sensoren verbunden und konfiguriert ist, um jeweilige Drucksignale zu erfassen und sie gemäß einer Abfolge von in Segmenten organisierten Proben abzutasten, und konfiguriert ist, um alle Schritte eines der Ansprüche 1 bis 12 auszuführen.

**Revendications**

1. Procédé de mesure en temps réel de la vitesse de l'onde sphygmique (PWV) acquise comme décalage temporel entre les pieds d'une même onde sphygmique acquise en deux sites différents d'un corps humain sous forme de signaux électriques correspondants générés par autant de capteurs (TS1, TS2) aptes à détecter une impulsion de pression au niveau d'une artère du corps humain, le procédé comprenant un processus d'acquisition (étape i) des deux signaux électriques et un processus d'analyse simultanée (AN) des deux signaux électriques en temps réel, dans lequel le processus d'analyse comprend la recherche (étape vb), pour chaque signal électrique, du pied (P) de l'onde sphygmique dans une portion (W) du signal non filtré ou filtré passe-haut (étape iv) correspondant à un voisinage (W) d'un minimum relatif de la même portion du signal filtré passe-bande et dans lequel ladite recherche est effectuée dans deux segments contigus (CS) du signal électrique au moyen d'une fenêtre d'observation glissante (T), contenue dans lesdits deux segments contigus, telle que pour toujours inclure une onde sphygmique entière.

2. Procédé selon la revendication 1, dans lequel ladite fenêtre d'observation glissante a une largeur variable et proportionnelle à une période calculée sur une succession d'événements déclencheurs identifiés.

3. Procédé selon la revendication 1 ou 2, dans lequel ledit processus d'analyse comprend

   - filtrage passe-bande du signal représenté dans au moins le segment courant,
   - identification des minima relatifs (starter d'événement) dans le signal filtré à l'étape précédente,
   - vérification si un voisinage (W) du starter d'événement est entièrement contenu dans le segment courant (CS), si oui
   - calcul du pied de l'onde sphygmique dans ledit voisinage (W) sur le signal passe-haut non filtré ou filtré, sinon, si ce voisinage (W) n'est pas entièrement contenu dans le segment courant
   - rattachement de la portion disponible du voisinage (W) à un prochain segment courant (CS) et
   - calcul du pied (P) de l'onde sphygmique dans ledit voisinage, sur le signal non filtré ou filtré passe-haut uniquement.

4. Procédé selon l'une quelconque des revendications précédentes, dans lequel si le voisinage (W) n'est pas entièrement contenu dans le segment courant, alors si un sous-intervalle (W') du voisinage ayant une amplitude prédéterminée est entièrement contenu dans le segment courant, les minima relatifs sont identifiés et mémorisés de sorte que lorsque le segment courant suivant est disponible, le minimum (MR) immédiatement précédent (gauche) de la tangente (Tan) est sélectionné.

5. Procédé selon l'une quelconque des revendications précédentes, comprenant une étape de construction d'un segment donné par le segment courant (CS) et par au moins un segment adjacent qui le précède avant de réaliser l'étape de filtrage passe-bande.

6. Procédé selon la revendication 5, dans lequel ledit filtrage passe-bande comprend un filtrage passe-haut et un filtrage passe-bas et dans lequel chacun d'eux comprend la mise en œuvre d'un filtre biquadratique du quatrième ordre.

7. Procédé selon la revendication 6, dans lequel ledit filtrage comprend les opérations suivantes de manipulation du signal successivement:

   - Inversion,
   - Premier filtrage,
   - Inversion,
   - Deuxième filtrage.

8. Procédé selon l'une quelconque des revendications précédentes, comprenant l'étape de calcul du paramètre PWV sur la base de la distance entre un premier capteur (TS1) associé à un premier site et un deuxième capteur (TS2) associé à un deuxième site, et sur la base d'un décalage temporel entre les pieds d'une même impulsion détectée dans le premier et le deuxième site.

9. Procédé selon la revendication 8, comprenant en outre une étape de calcul d'un écart type sur une fenêtre mobile des dernières valeurs du paramètre PWV et d'indication de la mesure comme fiable lorsque l'écart standard est inférieur à un seuil prédéterminé.

10. Procédé selon l'une quelconque des revendications précédentes, comprenant une étape de calcul d'un rapport signal sur bruit des signaux acquis par les capteurs (TS1, TS2) et d'indication de la mesure comme fiable lorsque ce rapport dépasse un deuxième seuil prédéterminé.

11. Procédé selon l'une quelconque des revendications précédentes, dans lequel le processus d'acquisition commence par des segments ayant une amplitude correspondant à une durée prédéterminée et comprend une étape de synchronisation dans laquelle l'amplitude et la phase de chaque segment sont synchronisées avec le signal de pression détecté par les capteurs, de sorte qu'une seule impulsion de pression soit contenue dans un segment.

**12.** Procédé selon la revendication 11, dans lequel ladite durée prédéterminée est de 1,5 seconde.

**13.** Programme informatique comprenant des moyens de codage de programme adaptés pour réaliser toutes les étapes de l'une quelconque des revendications 1 à 12, lorsque ledit programme est exécuté sur un ordinateur connecté de manière opérationnelle auxdits capteurs (TS1, TS2) agencés pour être associés dans deux sites différents du corps pour détecter une impulsion de pression.

**14.** Moyen lisible par ordinateur comprenant un programme enregistré, ledit moyen lisible par ordinateur comprenant un moyen de codage de programme adapté pour réaliser toutes les étapes de l'une quelconque des revendications 1 à 12, lorsque ledit programme est exécuté sur un ordinateur connecté de manière opérationnelle auxdits capteurs (TS1, TS2) agencés pour être associés à deux sites différents du corps pour détecter une impulsion de pression.

**15.** Système de mesure en temps réel de la vitesse de l'onde sphygmique (PWV) comprenant une paire de capteurs (TS1, TS2) adaptés pour détecter une impulsion de pression en correspondance avec une artère du corps humain, une unité de traitement connectée de manière opérationnelle à ladite paire de capteurs et configurée pour acquérir des signaux de pression respectifs et pour les échantillonner selon une succession d'échantillons organisés en segments et configurée pour réaliser toutes les étapes de l'une quelconque des revendications 1 à 12.

Fig. 1 (prior art)

Fig. 2

Fig. 3

EP 4 247 245 B1

Event
starter

CS

$\frac{T}{X}$  $\frac{T}{Y}$

W

DS

# Fig. 4

$\frac{T}{X}$  W'  $\frac{T}{3}$

Fig. 5

$\frac{T}{X}$  $\frac{T}{Y}$

W

Fig. 6

Fig. 7

*(i)* Array of 1.5 sec

*(ii)* Union with the previous 1.5 sec

*(iii)* Resampling

*(iv)* High pass filter

(v.a) Low pass filter

*(v)* (v.b) "Event starter" identification

(v.c) Period "T" evaluation

*(vi)* Local minimums identification    AN

*(vii)*
Data are stored in the "to append buffer"  — N — Is the T window included in the array?
Y
"to append buffer" is added — Y — Is the "to append buffer" not empty?
N

*(viii)* Intersecting tan algorithm

## Fig. 8

Fig. 9

**EP 4 247 245 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Local Pulse Wave Velocity: Theory, Methods, Advancements, and Clinical Applications. **NABEEL P M et al.** IEEE REVIEWS IN BIOMEDICAL ENGINEERING. IEEE, 26 July 2019, vol. 13